# DEMANDE DE BREVET EUROPEEN

(11) **EP 1 880 612 A1**
(43) Date de publication de la demande: **23.01.2008**
(21) Numéro de dépôt: 06117336.5
(22) Date de dépôt: 17.07.2006
(51) Int. Cl.: A23C 9/142

(54) **Ingrédient laitier enrichi en lipides polaires et applications de celui-ci**

(71) Demandeur: Corman S.A., 4834 Goe (BE)
(72) Inventeur: Dalemans, Daniel, 4040, Herstal (BE)
(74) Mandataire: pronovem

(57) **Abrégé**

Un ingrédient laitier enrichi en lipides polaires, en particulier en phospholipides et en sphingolipides dans lequel le pourcentage en phospholipides est supérieur à 10% en poids, par rapport au poids de matière sèche de l'ingrédient, son procédé d'obtention au départ de crème de lait pasteurisée et son application dans des produits alimentaires et/ou pharmaceutiques.

## Description

### Objet de l'invention

La présente invention est relative à un ingrédient laitier enrichi en composants de la membrane des globules gras du lait, c'est-à-dire enrichi en lipides polaires, en particulier en phospholipides et en sphingolipides.

La présente invention concerne également le procédé d'obtention d'un tel ingrédient laitier enrichi en composants de la membrane des globules gras du lait, à partir d'une crème de lait pasteurisée.

Le dernier aspect de la présente invention concerne également l'application avantageuse desdits ingrédients laitiers, à titre de compléments alimentaires, dans des compositions alimentaires ou dans des compositions pharmaceutiques.

### Arrière-plan technologique et état de la technique à la base de l'invention

Il est connu depuis de nombreuses années que les produits alimentaires ont des implications sur la santé du consommateur.

En particulier, l'excès de consommation de matières grasses dans l'alimentation humaine peut provoquer l'apparition de pathologies graves engendrées notamment par une augmentation du cholestérol et des triglycérides sanguins, plus spécifiquement le cholestérol.

Pour améliorer l'état de santé du consommateur, on a proposé des nouveaux produits allégés en matières grasses qui malheureusement ne présentent pas les mêmes propriétés organoleptiques que les produits standards.

Il a également été proposé d'utiliser des produits définis comme « alicaments » qui sont constitués par des produits alimentaires présentant un effet thérapeutique ou prophylactique dans le cadre d'un complément au traitement et/ou à la prévention de différentes pathologies (ostéoporose, maladies cardiovasculaires, cancer ...).

De tels ingrédients alimentaires sont par exemple constitués par des sucres, des protéines, des minéraux ou des vitamines susceptibles d'améliorer ou de maintenir la santé de l'homme.

Le document WO02/34062 décrit un procédé pour obtenir un produit enrichi en phospholipides et en sphingolipides par ultrafiltration sur une membrane présentant une valeur de cut-off comprise entre 5000 et 20000 Da et de préférence appauvri ou dépourvu de caséine.

Cette demande de brevet décrit également des produits alimentaires ou des suppléments alimentaires comprenant le produit enrichi en phospholipides et en sphingolipides obtenus.

La demande internationale de brevet W02003/071875 décrit un procédé de préparation d'un concentré enrichi en lipides polaires laitiers, en particulier un concentré enrichi en sphingolipides laitiers.

Ce dispositif décrit l'utilisation en parallèle de procédés d'ultrafiltration et de diafiltration au moyen de membranes de 30.000 ou de 10.000 Da. Ce procédé nécessite une étape préalable d'hydrolyse enzymatique des protéines de la matière première employée, à savoir un lactosérum de fromagerie ultrafiltré. Le procédé décrit permet d'obtenir un concentré enrichi en sphingolipides.

Cependant, le produit concentré obtenu présentera des propriétés organoleptiques détériorées par la présence des composés peptidiques issus de la protéolyse. En outre, le produit de départ employé pour le traitement n'est pas de la crème de lait, mais un lactosérum de fromagerie concentré par ultrafiltration et éventuellement par diafiltration (ce produit s'appelle aussi WPC pour Whey Protein Concentrate ou Procream qui est un nom commercial).

De plus, ce document décrit aussi une étape de concentration par ultrafiltration (en parallèle), qui est appliqué après l'hydrolyse enzymatique des protéines.
Cette hydrolyse enzymatique permet aux fractions de protéines (peptides) de traverser la membrane d'ultrafiltration et de se séparer de la fraction lipidique qui reste retenue par cette membrane.

### But de l'invention

La présente invention vise à obtenir un ingrédient laitier enrichi en composants lipidiques de la membrane du globule gras du lait, c'est-à-dire enrichi en lipides polaires, en particulier en phospholipides et en sphingolipides, mais comportant également et toujours les caractéristiques d'un ingrédient laitier, c'est-à-dire aussi essentiellement dépourvu de protéines hydrolysées, tout en maintenant ou en améliorant les propriétés organoleptiques typiques d'un ingrédient laitier.

La présente invention vise aussi à augmenter les propriétés organoleptiques ou structurelles de compositions alimentaires incorporant ces ingrédients et à apporter une dose journalière adéquate en ces dits lipides polaires, en particulier des sphingolipides, de manière à maintenir ou améliorer un état de santé adéquat du consommateur.

Un but particulier de la présente invention vise à permettre au consommateur d'obtenir une réduction du taux sanguin de cholestérol et des triglycérides, un effet préventif du cancer, en particulier du cancer du colon, à renforcer l'immunité et la flore intestinale du consommateur, à obtenir des effets antidiabétiques (traitement et/ou prévention du diabète) et à assurer une protection du foie du consommateur.

Un dernier but de l'invention vise à obtenir un procédé physique d'obtention de cet ingrédient laitier qui respecte les propriétés organoleptiques de l'ingrédient laitier obtenu.

### Eléments caractéristiques de l'invention

La présente invention est relative à un ingrédient laitier enrichi en composants de la membrane des globules gras du lait, c'est-à-dire enrichi en lipides polaires, en particulier en phospholipides et en sphingolipides, mais également appauvri en protéines, en particulier appauvri en, ou dépourvu de, caséine, tout en assurant que l'ingrédient laitier de l'invention garde les propriétés organoleptiques du produit laitier donc il est issu.

On entend par lipides polaires, les lipides portant une tête ou un groupe polaire à l'une de leurs extrémités. Les lipides polaires les plus fréquents sont les phospholipides comprenant des groupes d'acide phosphorique. Parmi ces lipides, on peut citer les phosphoglycérides, tels que le phosphatidylethanolamine, la phosphatidylcholine, la phosphatidylserine ou la phosphatidylinositol. Les sphingolipides comportent également une tête polaire, mais aucun groupe glycérol.

On distingue trois sous-classes de sphingolipides : les sphingomyélines, les cérébrosides et les gangliosides. Seules les sphingomyélines comportent un groupe d'acide phosphorique.

Les sphingolipides présents de manière principale dans le lait, sont la sphingomyéline (SPH, ou céramide phosphorylcholine), les glucosides céramides, les lactosyles céramides et les gangliosides.

Les phospholipides principalement présents sont la phosphatidylcholine (PC), la phosphatidylethanolamine (PE), la phosphatidylserine (PS) et le phosphatidylinositol (PI). Les autres lipides présents dans l'ingrédient laitier de l'invention sont des lipides neutres (triglycérides) et du cholestérol.

Les protéines présentes dans le produit de l'invention sont les protéines laitières, c'est à dire, principalement la caséine, la lactalbumine et la lactoglobuline.

L'objet de l'invention concerne un ingrédient laitier enrichi en ces lipides polaires, c'est-à-dire une composition dont la concentration en phospholipides, composants majoritaires des lipides polaires, est supérieure à 10% en poids, de préférence supérieure à 20% en poids, de préférence supérieure à 30% en poids, calculée sur le pourcentage en matières sèches de la composition.

Les autres composés présents dans la composition de l'invention sont de l'eau, des protéines, des hydrates de carbone, tels que du lactose, des vitamines solubles dans l'eau, des enzymes et des cendres (minéraux). Dans la suite du texte, on regroupe également le lactose et les cendres sous la définition extrait sec dégraissé non protéique.

L'ingrédient laitier de l'invention est caractérisé par sa concentration élevée en lipides polaires, en particulier en phospholipides et en sphingolipides, ainsi que par sa faible concentration relative en ses autres constituants laitiers à savoir, en lactose, en sels minéraux mais aussi en protéines qui sont également de préférence partiellement extraites au cours du procédé d'enrichissement en lipides polaires.

De préférence, le produit de l'invention comprend également un faible pourcentage en protéines, de préférence inférieur à 40%, plus particulièrement inférieur à 30% ; voire inférieur à 10%, (par rapport à la teneur en matière sèche de l'ingrédient). En outre la composition comporte également un certain pourcentage en Saccharides (Lactose), mais qui peuvent aussi être extrait de la composition.

De manière avantageuse, les protéines de la composition de l'invention sont présentés sous une forme non hydrolysée par l'action d'enzymes protéases et/ou peptidases, afin de ne pas induire des effets néfastes sur les caractéristiques organoleptiques du produit final (aliment, produit pharmaceutique,...) obtenu.

L'ingrédient laitier de l'invention peut être présent sous forme solide, de préférence obtenu par une évaporation (concentration thermique et séchage) de l'eau présente dans la composition de l'invention, ceci afin d'obtenir des produits plus stables, plus faciles à manipuler ou à doser et moins soumis à une dénaturation biologique.

Un autre aspect de la présente invention concerne le procédé d'obtention de l'ingrédient laitier enrichi en composants de la membrane des globules gras du lait, en particulier en lipides polaires, c'est-à-dire en phospholipides et en sphingolipides, de préférence, l'ingrédient laitier de l'invention.

Le procédé pour obtenir un produit enrichi en lipides polaires de l'invention, en particulier en phospholipides et en sphingolipides, est un procédé combinant différentes opérations unitaires qui sont des opérations classiques en laiterie et qui n'altèrent pas les propriétés organoleptiques des produits concentrés obtenus et des co-produits générés. Ce procédé comprend au moins les deux étapes de traitement successives suivantes :
- une concentration par centrifugation,
- une séparation sur membrane (ultrafiltration et/ou une combinaison d'ultrafiltration/diafiltration).

De préférence, le procédé de l'invention comprend au moins une concentration par centrifugation et une étape d'ultrafiltration/diafiltration de la composition enrichie de manière croissante en lipides polaires.

Selon une variante d'exécution du procédé de l'invention, celui-ci comprend une ou plusieurs étapes de concentration par centrifugation et plusieurs étapes d'ultrafiltration/diafiltration.

Selon une forme d'exécution préférée de l'invention, le procédé de l'invention comporte en outre une ou plusieurs étapes de lavage des extraits concentrés, par addition d'eau pure (non tamponnée), de préférence avant une nouvelle étape de concentration par centrifugation.

Avantageusement, le procédé de l'invention comporte également une ou plusieurs étapes (successives) dites de déprotéinisation et aptes à réduire très fortement la concentration des protéines initialement présentes.

De préférence, cette étape de déprotéinisation comporte un traitement thermo-calcique permettant la précipitation de la caséine et le séparation de celle-ci du milieu ; le traitement thermo-calcique comprend l'ajout d'environ 0.1 % de chlorure de calcium (W/W), suivi d'un traitement thermique à environ 70°C pendant environ 40 minutes, un ajustement du pH à environ 5.2 par ajout d'acide alimentaire (acide citrique ou acide lactique ou acide phosphorique ou acide chlorhydrique); la séparation ultérieure des protéines précipitées est réalisée par décantation centrifuge (au moyen d'un séparateur de la phase solide de la phase liquide).

Dans l'étape de traitement thermo-calcique de l'invention, l'addition d'acide citrique est préférée, mais cette étape de coagulation des protéines peut également être obtenue par l'action de la présure et des acides cités ci-dessus.

De préférence, dans le procédé de l'invention, la crème de lait pasteurisée est également soumise à un traitement thermique préalable, par exemple par un chauffage à une température comprise entre environ 60° C et environ 75°C, de préférence à une température comprise entre environ 65°C et environ 70°C, pendant une durée adéquate (environ 5 à environ 20 minutes, c'est-à-dire la durée de la centrifugation continue).

Un dernier aspect de la présente invention concerne une composition pharmaceutique (alicament), une composition alimentaire ou un additif alimentaire comprenant (outre les véhicules pharmaceutiques ou alimentaires adéquats) l'ingrédient laitier enrichi en lipides polaires, en particulier en phospholipides et en sphingolipides selon l'invention. Ladite composition alimentaire ou ledit additif pour composition alimentaire présente des propriétés organoleptiques ou structurelles équivalentes ou améliorées par rapport à des produits alimentaires standards, préparés sans cet ingrédient.

La composition pharmaceutique comprendra un véhicule pharmaceutique adéquat et ledit ingrédient (à titre de principe actif) en une proportion adéquate pour induire un effet thérapeutique ou préventif de certaines pathologies, (en particulier celles décrites dans les exemples).

La présente invention sera décrite de manière plus détaillée dans les exemples d'exécution ci-dessous présentés à titre d'illustrations non limitatives de l'invention en référence aux figures annexées.

### Description des figures

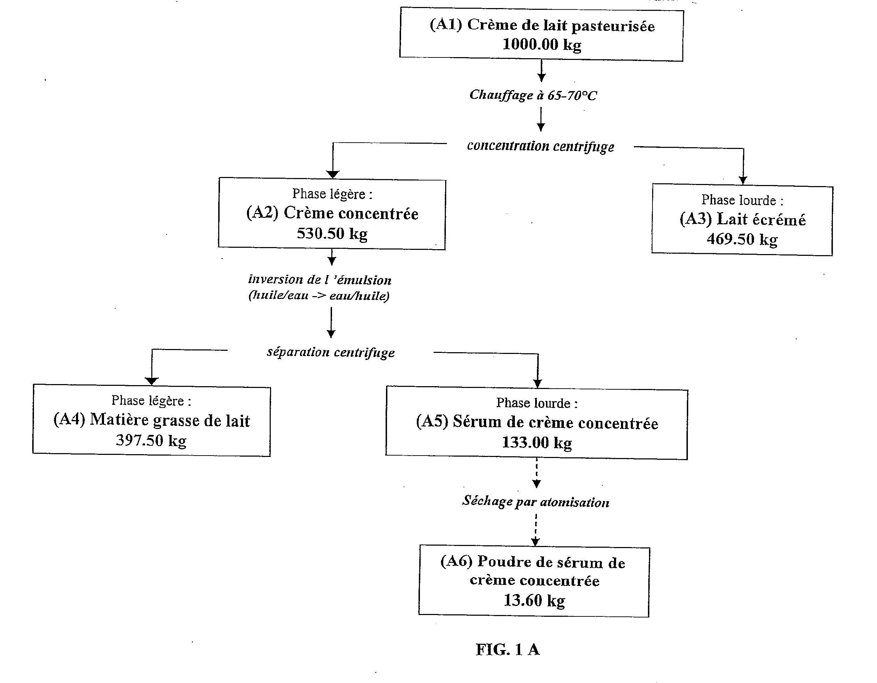

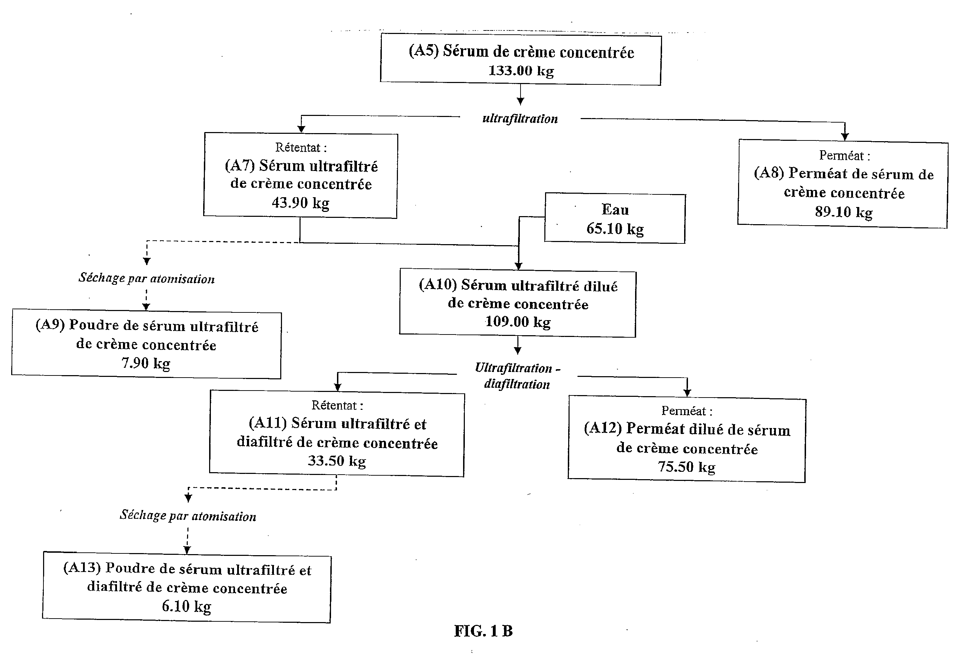

Les figures 1a et 1b représentent, de manière schématique, les différentes étapes du procédé de préparation de l'ingrédient laitier de l'invention à partir de crème de lait pasteurisée. Cet ingrédient est dénommé sérum de crème concentrée (figure 1a) et est avantageusement concentré par un traitement d'ultrafiltration / diafiltration (figure 1b).

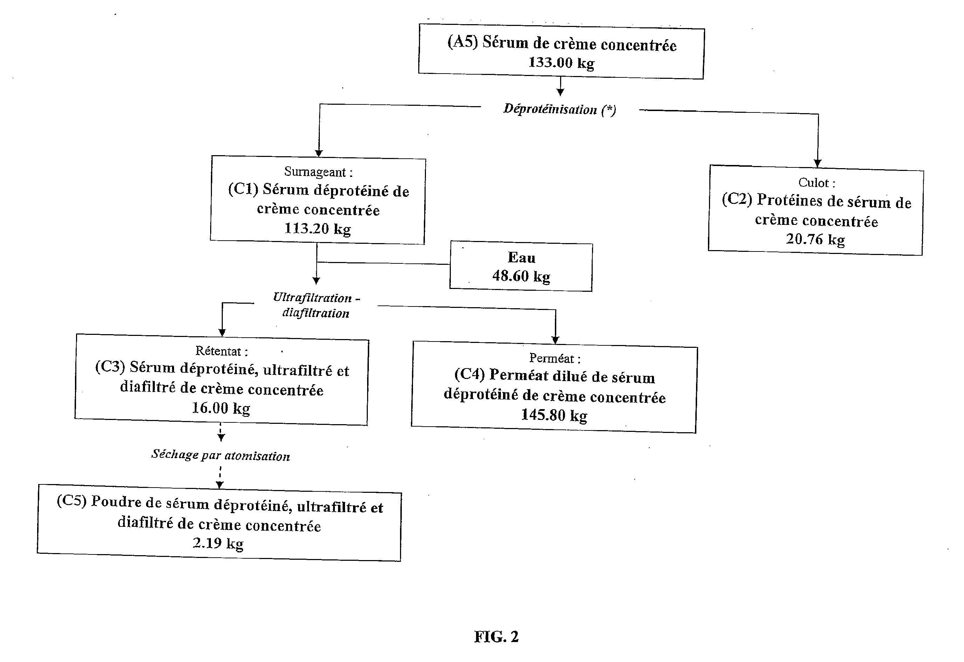

La figure 2 représente, de manière schématique, les étapes du procédé d'obtention de l'ingrédient laitier de l'invention à partir d'un sérum de crème concentrée obtenu par les traitements décrits à la figure 1a. Une étape de déprotéinisation et une étape de concentration par ultrafiltration et diafiltration permettent d'obtenir un sérum déprotéiné, ultrafiltré et diafiltré de crème concentrée.

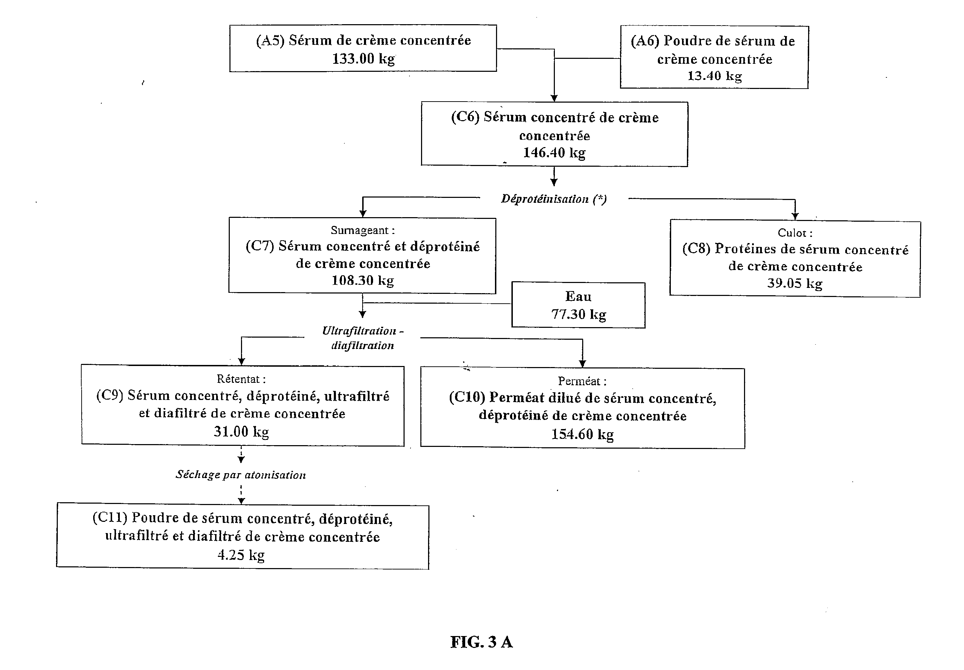

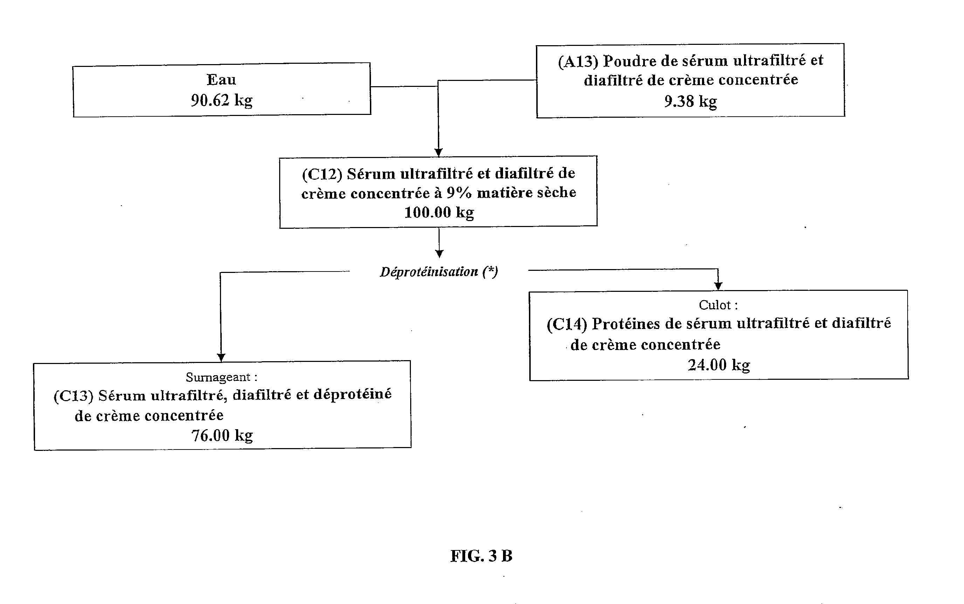

Les figure 3a et 3b représentent, de manière schématique, le même procédé que celui décrit à la figure 2 mais réalisé au départ d'un sérum de crème concentrée enrichi en extrait sec total ; ce procédé donne également un sérum concentré déprotéiné, ultrafiltré et diafiltré de crème concentrée, identique à celui obtenu par les traitements décrits à la figure 2, sans réduction du facteur de concentration des lipides polaires.

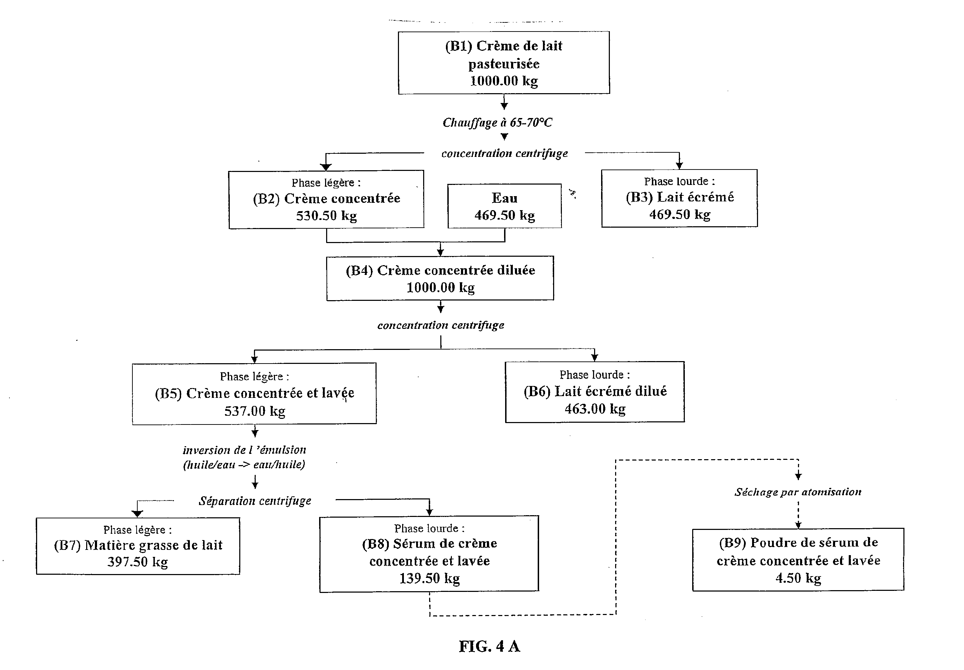

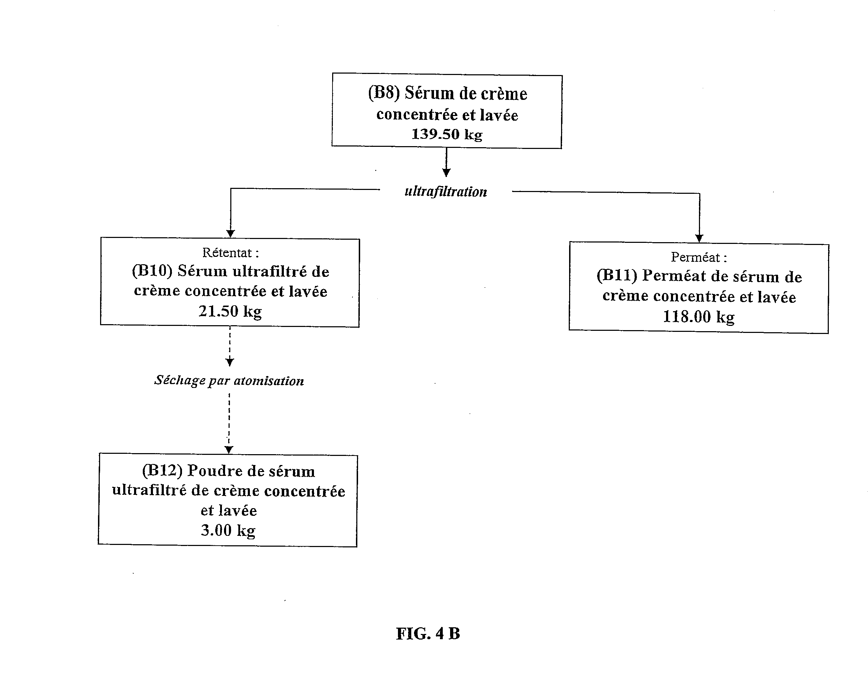

Les figures 4a et 4b représentent, de manière schématique, les différentes étapes du procédé d'obtention de l'ingrédient laitier de l'invention à partir d'une crème de lait concentrée et diluée à l'eau. Cet ingrédient est dénommé sérum de crème concentrée et lavée (figure 4a) et est avantageusement concentré par un traitement d'ultrafiltration et éventuellement de diafiltration (figure 4b).

### Description détaillée de l'invention

### Exemple 1

Tel que représenté à la figure 1a, il est possible d'obtenir de manière avantageuse l'ingrédient laitier de l'invention à partir d'une crème de lait pasteurisée (A1).

Environ 1000 kilos de crème de lait pasteurisée (A1) sont chauffés à une température comprise idéalement entre environ 65 et environ 70°C (pendant toute l'étape de centrifugation qui se réalise en continu ; cette durée est de l'ordre de 5 à 20 minutes).

La crème de lait ainsi chauffée est soumise à une première centrifugation qui permet de concentrer une phase légère (A2) de crème concentrée et une phase lourde (A3) de lait écrémé. La proportion de la phase légère et de la phase lourde est essentiellement équivalente (environ 530 kilos de la phase légère (A2) et environ 470 kilos d'une phase lourde (A3)).

La phase légère constituée de crème concentrée (A2) sous forme d'une émulsion « huile dans eau » est ensuite transformée en une émulsion « eau dans huile » par une étape dite d'inversion de l'émulsion ; cette étape est suivie d'une deuxième centrifugation permettant de re-concentrer la phase légère de crème (A2) en une nouvelle phase légère (A4) et une nouvelle phase lourde (A5).

La phase dite légère (A4) comprend essentiellement les matières grasses du lait ; la phase lourde constituant un sérum de crème concentrée (A5).

La proportion entre cette phase légère (A4) et cette phase lourde (A5) est d'environ 3/4 - 1/4. (environ 397 kilos de phase légère de matières grasses de lait (A4) et environ 133 kilos de phase lourde (sérum de crème concentrée (A5)).

Ensuite, la phase lourde (A5) est soumise de manière avantageuse à une étape de séchage fournissant un sérum de crème concentrée sous une forme solide (A6) (poudre de sérum de crème concentrée) pour un poids d'environ une quatorze kilos (13,6 kilos).

Selon une première variante d'exécution de l'invention (figure 1b), on récupère et traite ladite phase lourde constituée de sérum de crème concentrée (A5) par une étape supplémentaire comprenant une ultrafiltration permettant de récupérer à la fois un rétentat et un perméat.

Le rétentat est constitué d'un sérum ultrafiltré de crème concentrée (A7) et le perméat est constitué d'un perméat de sérum de crème concentrée (A8). La proportion entre le rétentat et le perméat est d'environ 1/3 - 2/3 (environ 44 kilos de rétentat et environ 89 kilos de perméat).

Selon une alternative de l'invention, il est également possible d'effectuer un séchage direct du sérum ultrafiltré de crème concentrée (A7) pour obtenir un produit solide : une poudre de sérum ultrafiltré de crème concentrée (A9). La quantité de poudre obtenue est d'environ 8 kilos (7,90 kilos).

Selon une autre forme d'exécution préférée de l'invention, le sérum ultrafiltré de crème concentrée (A7) est dilué avec de l'eau, pour former un sérum ultrafiltré dilué de crème concentrée (A10) qui est à nouveau concentré par ultrafiltration (diafiltration).

Cette dilution s'effectue par environ 65 kilos d'eau (i.e. 65,10 kg) ajoutée à environ 44 kilos (43,90 kg) de sérum ultrafiltré de crème concentrée (A7) pour former une solution d'un sérum ultrafiltré dilué de crème concentrée (A10) d'un poids total de 109 kilos.

Le sérum ultrafiltré dilué de crème concentrée (A10) est soumis à une seconde étape d'ultrafiltration (diafiltration), de manière à obtenir un nouveau rétentat (A11) et un nouveau perméat (A12), le rétentat étant constitué d'un sérum ultrafiltré et diafiltré de crème concentrée (A11) et le perméat étant constitué d'un perméat dilué de sérum de crème concentrée (A12).

La proportion entre le rétentat (A11) et le perméat (A12) est d'environ 30/70 (35,50 kilos de rétentat (A11) et 75,50 kilos de perméat (A12)).

Le rétentat (A11) peut être à nouveau soumis à une étape de séchage pour obtenir un produit solide: une poudre de sérum ultrafiltré et diafiltré de crème concentrée (A13). Le poids total de cette poudre est d'environ 6 kilos (6, 10 kilos).

La table 1 présente les caractéristiques de composition des différents produits obtenus après les différentes étapes réalisée selon le procédé décrit ci-dessus.

La table 1 présente, en pourcentage en poids, les teneurs en eau, en matière grasse totale, en phospholipides (majeure partie des lipides polaires, compris dans la matière grasse totale), en extrait sec dégraissé et les constituants de cet extrait sec dégraissé à savoir, protéines, lactose et cendres.

On observe que le sérum de crème concentrée (A5) soumis aux étapes supplémentaires d'ultrafiltration et de diafiltration est enrichi en composants de la membrane des globules gras du lait, c'est-à-dire en lipides polaires (phospholipides), ainsi qu'en lipides totaux (dont les lipides neutres) et en protéines ; Cet enrichissement se produit essentiellement au détriment des teneurs en lactose surtout et en cendres dans une moindre mesure.

Les produits obtenus sont des ingrédients laitiers riches en lipides polaires. Ces ingrédients laitiers contiennent respectivement plus de 7%, plus de 11% et plus de 14% de phospholipides laitiers, ces pourcentages étant exprimés par rapport à la matière sèche.

De manière avantageuse, l'ingrédient laitier de l'invention est présenté sous forme solide et est obtenu par un séchage d'un sérum de crème concentrée provenant d'une concentration thermique et d'un séchage, de préférence par atomisation.

Selon une seconde forme d'exécution préférée de l'invention représentée à la figure 2, 133 kg de sérum de crème concentrée (A5) est soumis à une étape d'extraction des protéines (« déprotéinisation ») par traitement dit « thermo-calcique » comprenant l'ajout de 0,1% (en poids) de chlorure de calcium , suivi d'un chauffage à une température d'environ 70°C, d'un ajustement du pH du sérum à environ 5.2 par adjonction d'acide citrique et un maintien du sérum à cette température pour une durée d'environ 40 minutes).

Ensuite, les protéines précipitées sont extraites par une étape supplémentaire de décantation centrifuge (séparateur solide-liquide). On obtient ainsi un culot d'environ 21 kg (20,76 kg) comprenant les protéines de sérum de crème concentrée (C2) et un surnageant d'environ 113 kg (113,20 kg) de sérum déprotéiné de crème concentrée (C1).

Celui-ci est ensuite soumis à une ultrafiltration/diafiltration, avec dilution au moyen d'environ 49 kg (48,6 kg) d'eau. Cette opération fournit 16 kg de rétentat constitué d'un sérum déprotéiné, ultrafiltré et diafiltré de crème concentrée (C3) et environ 146 kg (145,80 kg) d'un perméat dilué de sérum déprotéiné de crème concentrée (C4).

Le rétentat est soumis à une étape supplémentaire de séchage (par atomisation) pour obtenir environ 2,2 kg (2,19 kg) de poudre de sérum déprotéiné, ultrafiltré et diafiltré de crème concentrée (C5). Les caractéristiques des produits obtenus sont reprises sur la table 2. Le sérum déprotéiné, ultrafiltré et diafiltré de crème concentrée constitue un ingrédient laitier fortement enrichi en lipides polaires du lait ; sa teneur en phospholipides est de plus de 35% en poids, ce pourcentage étant exprimé par rapport à la matière sèche.

Selon une troisième forme d'exécution préférée de l'invention représentée à la figure 3a, le sérum de crème concentrée (A5) (133 kg) peut être combinée à de la poudre de sérum concentrée (A6) (13,4 kg) pour obtenir environ 146 kg (146,4 kg) d'un sérum de crème concentrée (C6) à plus haute teneur en matière sèche.

Ce produit est soumis à une étape de déprotéinisation telle que décrite ci-dessus pour obtenir un surnageant d'environ 108 kg (108,3 kg) constitué d'un sérum concentré et déprotéiné de crème concentrée (C7) et d'un culot d'environ 39 kg (39,05 kg) constitué de protéines de sérum concentré d'une crème concentrée (C8).

Le surnageant est ensuite soumis à une étape d'ultrafiltration/diafiltration avec addition d'environ 77 kg d'eau (77,3 kg) pour obtenir environ 31 kg d'un rétentat constitué de sérum concentré, déprotéiné ultrafiltré et diafiltré de crème concentrée (C9) et environ 154 kg (154,6 kg) d'un permeat dilué d'un sérum concentré, déprotéiné de crème concentrée (C10). Le rétentat peut être soumis avantageusement à une étape supplémentaire de séchage par atomisation pour obtenir un peu plus de 4 kilos (4,25 kg) d'une poudre de sérum concentré, déprotéiné, ultrafiltré et diafiltré de crème concentrée (C11). Les caractéristiques des produits obtenus sont représentées dans la table 3. A nouveau, le sérum concentré, déprotéiné, ultrafiltré et diafiltré de crème concentrée obtenu constitue un ingrédient laitier fortement enrichi en lipides polaires du lait ; sa teneur en phospholipides est de plus de 35% en poids, ce pourcentage étant exprimé par rapport à la matière sèche. L'augmentation de la matière sèche du sérum de crème concentrée n'a pas affecté le facteur de concentration des lipides polaires récupérés après déprotéinisation et ultrafiltration/diafiltration.

De manière alternative, il est également possible de traiter la poudre de sérum ultrafiltré/diafiltré de crème concentré (A13) (9,38 kg) par addition d'eau (90,62 kg) pour obtenir 100 kg d'un sérum ultrafiltré/diafiltré de crème concentrée à 9 % de matière sèche (C12). Ce produit est soumis à une étape de déprotéinisation (figure 3b) telle que décrite ci-dessus, pour obtenir 76 kg d'un surnageant constitué d'un sérum ultrafiltré, diafiltré et déprotéiné de crème concentrée (C13) et 24 kg d'un culot constitué de protéines de sérum ultrafiltré, diafiltré de crème concentrée (C14). Les caractéristiques de ces derniers produits sont représentées dans la table 4. Le sérum ultrafiltré, diafiltré et déprotéiné de crème concentrée (C13) constitue l'ingrédient laitier concentré en lipides polaires de ce procédé ; sa teneur en phospholipides laitiers est de 0,95%, soit plus de 20% en poids exprimé sur la matière sèche.

### Exemple 2

Tel que représenté à la figure 4a, il est possible d'obtenir également l'ingrédient laitier de l'invention, selon une variante d'exécution de celle représentée à l'exemple 1. Environ 1000 kg de crème de lait pasteurisée (B1) sont soumis à un traitement thermique, similaire à l'exemple 1 et ensuite à une première centrifugation, similaire à celle de l'exemple 1, pour obtenir une phase légère de crème concentrée (B2) et une phase lourde de lait écrémé (B3). Les proportions sont équivalentes à celles de l'exemple 1. La phase légère (B2) est diluée par une quantité similaire d'eau pure (non tamponnée) pour obtenir une crème concentrée diluée (B4) (environ 1000 kg). Cette crème concentrée diluée (B4) est soumise à une seconde concentration par centrifugation de manière à obtenir une phase légère constituée d'une crème concentrée et lavée (B5) et d'une phase lourde constituée d'un lait écrémé dilué (B6) (environ 537 kg de crème concentrée et lavée (B5) et environ 463 kg de lait écrémé dilué (B6)).

La crème concentrée et lavée (B6) est ensuite soumise à une étape d'inversion de l'émulsion et à une nouvelle concentration par centrifugation, telle que décrite dans l'exemple 1. On obtient ainsi à partir de la crème concentrée et lavée (B5) une phase légère constituée d'une matière grasse de lait (B7) (environ 396 kg) et une phase lourde, constituée d'un sérum de crème concentrée et lavée (B8) (environ 140 kg). Ce sérum de crème concentrée et lavée peut être soumis à une étape de séchage, telle que décrite dans l'exemple 1 pour obtenir une poudre de sérum de crème concentrée et lavée (B9) pour un poids total d'environ 4,50 kg.

Ledit sérum de crème concentrée et lavée (B8) peut être à nouveau soumis à une étape d'ultrafiltration, présentée à la figure 4b et telle que déjà décrite pour l'exemple 1, de manière à obtenir environ 21,50 kg de rétentat constitué d'un sérum ultrafiltré de crème concentrée et lavée (B10) et environ 118 kg de perméat de sérum de crème concentrée et lavée (B11). Le rétentat (sérum ultrafiltré de crème concentrée et lavée (B10)) peut être également soumis à une nouvelle étape de séchage, tel que décrit dans l'exemple 1 pour obtenir un produit sous forme solide constitué d'une poudre de sérum ultrafiltré de crème concentrée et lavée (B12) d'un poids total d'environ 3,00 kg.

La table 5 présente les caractéristiques de composition des produits obtenus selon le procédé de l'exemple 2 illustré dans les figures 4a et 4b. Les paramètres analysés sont les mêmes que ceux présentés dans la table 1. Le sérum de crème concentrée et lavée (B8) et le sérum ultrafiltré de crème concentrée et lavée (B10) sont des ingrédients laitiers fortement enrichis en lipides polaires de la membrane des globules gras du lait. La teneur en phospholipides laitiers de ces produits est respectivement de 0,62% et 3,90%, soit respectivement plus de 19% et plus de 27% exprimés sur la matière sèche. La concentration en lipides polaires est réalisée au détriment de l'extrait sec dégraissé pour l'opération de lavage de la crème et au détriment de l'extrait sec dégraissé non protéique pour l'ultrafiltration.

De manière avantageuse, les produits suivant, obtenus lors des procédés décrits ci-dessus dans les exemples 1 et 2, :
- sérum de crème concentrée,
- sérum ultrafiltré et éventuellement diafiltré de crème concentrée,
- sérum déprotéiné, ultrafiltré et éventuellement diafiltré de crème concentrée,
- sérum de crème concentrée et lavée, et
- sérum ultrafiltré de crème concentrée et lavée, enrichis en lipides polaires de la membrane des globules gras du lait (phospholipides et sphingolipides), sous leur forme liquide et/ou éventuellement sous leur forme solide, peuvent être utilisés dans une composition alimentaire et être incorporés avec les composants alimentaires habituels d'une composition alimentaire destinée à l'humain ou à l'animal.

Il est ainsi possible d'obtenir des compositions alimentaires (éventuellement allégées en certaines matières grasses, néfastes pour la santé, en particulier allégées en lipides non polaires), tout en maintenant les propriétés organoleptiques des produits standard ou offrant des propriétés organoleptiques améliorées par rapport aux produits standard.

L'ingrédient alimentaire de l'invention, caractérisé par un effet émulsifiant, peut être utilisé pour améliorer l'onctuosité des produits alimentaires, notamment des produits laitiers (allégés ou non en matières grasses), tels que des crèmes, des yoghourts, des yoghourts à boire, des fromages, en particulier des fromages à tartiner ou des desserts laitiers.

En outre, l'ingrédient alimentaire permet une meilleure rétention d'eau dans les produits de boulangerie/pâtisserie (pains au lait et brioches) améliorant ainsi la conservation du moelleux de ces produits par rapport à des produits standards préparés sans cet ingrédient alimentaire. En effet, les inventeurs ont observés de manière inattendue une amélioration de la rétention de l'eau dans les produits obtenu ce qui permet une meilleure conservation du moelleux des produits en diminuant leur séchage.

L'ingrédient laitier de l'invention peut être également utilisé pour améliorer l'aptitude au fouettage d'une crème laitière entière et allégée ayant ou non subi un traitement thermique de stérilisation UHT et laquelle l'ingrédient laitier de l'invention a été incorporé.
On observe également un effet émulsifiant naturel du lait favorisant les émulsions de type huile dans eau obtenu avec le produit sous forme de poudre dispersée dans de l'eau ou avec le produit sérum liquide de l'invention.

En outre, l'ingrédient laitier de l'invention présente une concentration élevée en lipides polaires, en particulier en phospholipides et en sphingolipides permettant d'améliorer de manière significative l'état de santé d'un patient, ou le maintien de l'état de santé du patient consommant directement cet ingrédient ou un produit alimentaire contenant cet ingrédient (H. Vesper et al. American Society for Nutritional Sciences, p. 1239-1250 (1999)).

Cet ingrédient enrichi en sphingolipides (sphingomyéline,...) permet avantageusement d'obtenir une réduction du taux de cholestérol sanguin VLDL et LDL et des triglycérides, un effet préventif du cancer, en particulier du cancer du colon, permet de renforcer l'immunité et la flore intestinale, en particulier de provoquer un effet prébiotique, c'est-à-dire favoriser la croissance d'une flore intestinale bénéfique (germes de type Bifidus notamment) par rapport à une flore intestinale pathogène et prévenir ou traiter des troubles digestifs (diarrhées).

En outre, l'ultra concentration dans l'ingrédient laitier des phospholipides et des sphingolipides peut avoir des effets anti-diabétiques (traitement et/ou prévention du diabète) et assurer la protection du foie.

Un dernier aspect de l'invention concerne une composition pharmaceutique (alicament ou « functional food ») comprenant un véhicule pharmaceutique adéquat et l'ingrédient laitier de l'invention, en particulier destiné au traitement et/ou à la prévention des pathologies susmentionnées, ainsi que un procédé de traitement préventif ou thérapeutique de l'une des pathologies susmentionnées chez un mammifère (y compris l'homme) dans lequel une quantité suffisante de cette composition est administrée à un mammifère (y compris l'homme) susceptible de souffrir de ces pathologies.

**Tableau 1**

| % en poids | Eau | Matière grasse totale | dont Phospholipides | Extrait sec dégraissé | dont Protéines | dont Lactose | dont Cendres |
|---|---|---|---|---|---|---|---|
| (A1) Crème de lait pasteurisée | 54.90% | 40.00% | 0.22% | 5.10% | 1.83% | 2.84% | 0.42% |
| (A2) Crème de lait concentrée | 22.88% | 75.00% | 0.35% | 2.13% | 0.76% | 1.18% | 0.18% |
| (A3) Lait écrémé | 91.13% | 0.40% | 0.07% | 8.47% | 3.04% | 4.72% | 0.71% |
| (A4) Matière grasse de lait | 0.37% | 99.60% | 0.23% | 0.03% | 0.01% | 0.02% | 0.00% |
| (A5) Sérum de crème concentrée | 90.17% | 1.45% | 0.75% | 8.38% | 3.01% | 4.67% | 0.70% |
| (A6) Poudre de sérum de crème concentrée | 4.00% | 14.17% | 7.33% | 81.83% | 29.40% | 45.62% | 6.82% |
| (A7) Sérum ultrafiltré de crème concentrée | 82.69% | 4.24% | 2.10% | 13.07% | 8.30% | 3.60% | 1.17% |
| (A9) Poudre de sérum ultrafiltré crème concentrée | de 4.00% | 23.51% | 11.65% | 72.49% | 46.03% | 19.97% | 6.49% |
| (A11) Sérum ultrafiltré et diafiltré de crème concentrée | 82.46% | 5.00% | 2.75% | 12.54% | 10.70% | 0.70% | 1.14% |
| (A13) Poudre de sérum ultrafiltré diafiltré et de crème concentrée | 4.00% | 27.40% | 15.00% | 68.60% | 58.53% | 3.83% | 6.24% |

**Tableau 2**

| % en poids | Eau | Matière grasse totale | dont Phospholipides | Extrait sec dégraissé | dont Protéines | dont Lactose | dont Cendres |
|---|---|---|---|---|---|---|---|
| (A5) Sérum de crème concentrée | 90.17% | .45% | 0.75% | 8.38% | 3.01% | 4.67% | 0.70% |
| (C1) Sérum déprotéiné de crème concentrée | 92.08% | 1.43% | 0.78% | 6.49% | 0.90% | 4.73% | 0.86% |
| (C2) Protéines de sérum de crème concentrée | 79.62% | 1.45% | 0.72% | 18.93% | 14.00% | 4.10% | 0.83% |
| (C3) Sérum déprotéiné, ultrafiltré et diafiltré de crème concentrée | 86.80% | 8.70% | 5.20% | 4.50% | 3.60% | 0.65% | 0.25% |
| (C4) Perméat dilué de sérum déprotéiné de crème concentrée | 95.47% | <0.10% | <0.10% | 4.50% | 0.25% | 3.60% | 0.65% |
| (C5) Poudre de sérum déprotéiné, ultrafiltré et diafiltré de crème concentrée | 3.50% | 63.60% | 38.00% | 32.90% | 26.30% | 4.76% | 1.84% |

**Tableau 3**

| % en poids | Eau | Matière grasse totale | dont Phospholipides | Extrait sec dégraissé | dont Protéines | dont Lactose | dont Cendres |
|---|---|---|---|---|---|---|---|
| (C6) Sérum concentré de crème concentrée | 82.30% | 2.60% | 1.35% | 15.10% | 5.30% | 8.51% | 1.29% |
| (C7) Sérum concentré et déprotéiné de crème concentrée | 85.90% | 2.60% | 1.45% | 11.50% | 1.35% | 8.75% | 1.40% |
| (C8) Protéines de sérum concentré de crème concentrée | 72.20% | 2.50% | 1.00% | 25.30% | 16.00% | 7.90% | 1.40% |
| (C9) Sérum concentré, déprotéiné, ultrafiltré et diafiltré de crème concentrée | 86.75% | 8.80% | 5.30% | 4.45% | 3.60% | 0.60% | 0.25% |
| (C10) Perméat dilué de sérum concentré, déprotéiné de crème concentrée | 92.83 | < 0.10% | <0.10% | 7.15% | 0.25% | 6.00% | 0.90% |
| (C11) Poudre de sérum concentré, déprotéiné, ultrafiltré et diafiltré de crème concentrée | 3.00% | 64.42% | 38.80% | 32.58% | 26.35% | 4.39% | 1.83% |

**Tableau 4**

| % en poids | Eau | Matière grasse totale | dont Phospholipides | Extrait sec dégraissé | dont Protéines | dont Lactose | dont Cendres |
|---|---|---|---|---|---|---|---|
| (A13) Poudre de sérum ultrafiltré et diafiltré de crème concentrée | 4.00% | 27.40% | 15.00% | 68.60% | 58.53% | 3.83% | 6.24% |
| (C12) Sérum ultrafiltré et diafiltré de crème concentrée | 91.00% | 2.57% | 1.40% | 6.43% | 5.48% | 0.36% | 0.59% |
| (C13) Sérum ultrafiltré, diafiltré et déprotéiné de crème concentrée à 9% matière sèche | 95.58% | 1.85% | 0.95% | 2.57% | 1.59% | 0.38% | 0.60% |
| (C14) Protéines de sérum ultrafiltré et diafiltré de crème concentrée | 76.75% | 4.30% | 2.70% | 18.95% | 18.00% | 0.35% | 0.60% |

**Tableau 5**

| % en poids | Eau | Matière grasse totale | dont Phospholipides | Extrait sec dégraissé | dont Protéines | dont Lactose | dont Cendres |
|---|---|---|---|---|---|---|---|
| (B1) Crème de lait pasteurisée | 54.90% | 40.00% | 0.22% | 5.10% | 1.83% | 2.84% | 0.42% |
| (B2) Crème de lait concentrée | 22.88% | 75.00% | 0.35% | 2.13% | 0.76% | 1.18% | 0.18% |
| (B3) Lait écrémé | 91.13% | 0.40% | 0.07% | 8.47% | 3.04% | 4.72% | 0.71% |
| (B4) Crème concentrée diluée | 59.09% | 39.79% | 0.19% | 1.13% | 0.40% | 0.63% | 0.09% |
| (B5) Crème concentrée et lavée | 25.52% | 74.00% | 0.32% | 0.48% | 0.17% | 0.27% | 0.04% |
| (B6) Lait écrémé dilué | 98.05% | 0.15% | 0.03% | 1.80% | 0.65% | 1.00% | 0.15% |
| (B7) Matière grasse de lait | 0.39% | 99.60% | 0.22% | 0.01% | 0.00% | 0.01% | 0.00% |
| (B8) Sérum de crème concentrée et lavée | 96.90% | 1.30% | 0.62% | 1.80% | 0.65% | 1.00% | 0.15% |
| (B9) Poudre de sérum de crème concentrée et lavée | 4.00% | 40.26% | 19.20% | 55.74% | 20.02% | 31.07% | 4.64% |
| (B10) Sérum ultrafiltré de crème concentrée et lavée | 86.50% | 8.00% | 3.90% | 5.50% | 4.30% | 0.60% | 0.60% |
| (B12) Poudre de sérum ultrafiltré de crème concentrée et lavée | 4.00% | 56.89% | 27.73% | 39.11% | 30.58% | 4.27% | 4.27% |

## Revendications

1. Un ingrédient laitier enrichi en lipides polaires, en particulier en phospholipides et en sphingolipides dans lequel le pourcentage en phospholipides est supérieur à 10% en poids, par rapport au poids de matière sèche de l'ingrédient.

2. L'ingrédient laitier selon la revendication 1, dans lequel le pourcentage en lipides polaires de type phospholipides est supérieur à 20%, de préférence 30% par rapport au poids de matière sèche de la composition.

3. L'ingrédient laitier selon l'une quelconque des revendications précédentes 1 ou 2, qu'il comporte en outre au moins un des éléments choisis parmi le groupe constitué par des protéines, des hydrates de carbone, des minéraux, des vitamines solubles dans l'eau, des enzymes et éventuellement des cendres.

4. L'ingrédient laitier selon la revendication 3, dans lequel le pourcentage en protéines est inférieur à 40%, plus particulièrement inférieur à 30%, voire inférieur à 10% par rapport à la teneur et matière sèche de l'ingrédient laitier.

5. L'ingrédient laitier selon la revendication 3 ou 4, dans lequel les protéines ne sont pas des protéines hydrolysées par des enzymes.

6. L'ingrédient laitier selon l'une quelconque des revendications précédentes, qui est sous forme liquide.

7. L'ingrédient laitier selon l'une quelconque des revendications précédentes 1 à 5, qui est sous forme solide (poudre).

8. Un procédé d'obtention d'un ingrédient laitier enrichi en lipides polaires, en particulier en phospholipides et en sphingolipides, dans lequel une crème de lait pasteurisée est soumise à au moins deux étapes de traitement physique, choisies parmi le groupe constitué par une concentration par centrifugation, une étape d'ultrafiltration et une étape d'ultrafiltration / diafiltration.

9. Le procédé selon la revendication 8, **caractérisé en ce qu'**il comprend en outre une étape d'extraction des protéines de l'ingrédient laitier.

10. Le procédé selon la revendication 9 dans le quel l'extraction des protéines est obtenue par un traitement thermocalcique comprenant l'ajout de chlorure de calcium, suivit d'un traitement thermique à 70°C pendant 40 minutes, d'un ajustement du PH à 5,2 par ajout d'acide et d'une séparation des protéines précipitées par décantation centrifuge.

11. Le procédé selon l'une quelconques des revendications 10, **caractérisé en ce que** l'acide est de l'acide citrique et/ou de l'acide lactique et/ou de l'acide phosphorique et/ou de l'acide chlorhydrique.

12. Le procédé selon la revendication 8 à 11 **caractérisé en ce qu'**il comprend en outre une étape de lavage par addition d'eau, de préférence une étape de lavage avant une étape de concentration par centrifugation.

13. Une composition alimentaire ou additif alimentaire, comprenant l'ingrédient laitier selon l'une quelconque des revendications précédentes 1 à 7.

14. La composition alimentaire selon la revendication 11, **caractérisée en ce qu'**elle est choisie parmi le groupe constitué par les produits laitiers, tels que des crèmes, des yoghourts, des yoghourts à boire, des fromages, des fromages à tartiner, des desserts laitiers, des soupes, des produits de boulangerie et de pâtisserie, en particulier des brioches ou des pains au lait.

15. Une composition pharmaceutique comprenant un véhicule pharmaceutique adéquat et l'ingrédient laitier, selon l'une quelconque des revendications précédentes 1 à 7.

16. Utilisation de la composition pharmaceutique selon la revendication 15 pour la préparation d'un médicament dans le traitement ou la prévention du cancer ou du diabète.
